# FASCICULE DE BREVET EUROPEEN

(11) **EP 1 126 821 B1**
(45) Date de publication et mention de la délivrance du brevet: **27.04.2005**
(21) Numéro de dépôt: 99954046.1
(22) Date de dépôt: 03.11.1999
(51) Int. Cl.: A61K 9/00, A61K 9/20

(54) **COMPRIME A DELITEMENT RAPIDE PERFECTIONNE**
VERBESSERTE TABLETTE MIT SCHNELLER AUFLÖSBARKEIT
IMPROVED FAST DISINTEGRATING TABLET

(30) Priorité: 06.11.1998 FR 9814034
(43) Date de publication de la demande: 29.08.2001
(73) Titulaire: ETHYPHARM, 78550 Houdan (FR)
(72) Inventeur: CHAUVEAU, Charles, F-06560 Valbonne (FR); ZUCCARELLI, Jean-Marc, 06600 Antibes (FR); NOURI, Nourredine, F-06400 Cannes (FR); BARBERO, Maryvonne, 06600 Antibes (FR)
(74) Mandataire: Touati, Catherine
(86) Numéro de dépôt international: PCT/FR1999/002681
(87) Numéro de publication internationale: WO 2000/027357

(56) Documents cités:
- EP-A- 0 745 382
- WO-A-89/02266
- WO-A-91/15194
- WO-A-93/01805
- US-A- 5 084 278
- US-A- 5 215 755
- US-A- 5 320 848
- US-A- 5 814 332

## Description

L'invention a pour objet un comprimé à délitement rapide du genre de ceux qui se désagrègent dans la bouche en moins de 40 secondes, lequel comprimé comprend des particules de principe actif enrobé, lesdites particules présentant des caractéristiques de compression intrinsèques, et un mélange d'excipients.

Comme principe actif pouvant servir à la réalisation des comprimés selon l'invention, on peut citer par exemple l'ibuprofène, le paracétamol et l'aspirine.

On connaît déjà des comprimés à base d'ibuprofène.

Ainsi, le brevet US 5,215,755 décrit des comprimés à mâcher dans lesquels l'ibuprofène est présent sous la forme de granules comportant un enrobage à base d'hydroxyéthylcellulose ou d'un mélange hydroxyéthylcellulose/hydroxypropylméthylcellulose. Cet enrobage a été choisi pour remédier aux déficiences constatées en rapport avec les enrobages antérieurs à base d'éthylcellulose seule.

EP-A-0745 382 décrit des comprimés à désagrégation rapide (1à 40s) qui peuvent contenir des particules de principe actif enrobé.

L'invention a pour but de fournir des comprimés obtenus à l'aide de particules de principe actif enrobé qui présentent non seulement un délitement rapide dans la bouche en moins de 40 secondes mais également une palatabilité agréable, ainsi que des caractéristiques de dureté satisfaisantes permettant l'industrialisation de sa fabrication et qui se conservent suffisamment dans des conditions normales de stockage pour permettre leur manipulation par le patient, ces comprimés permettant également une biodisponiblité optimale du principe actif.

Le comprimé conforme à l'invention est caractérisé par le fait qu'il est à base de particules de principe actif enrobé, lesdites particules présentant des caractéristiques de compression intrinsèques et d'un mélange d'excipients, la proportion de mélange d'excipients par rapport au principe actif enrobé étant de 0,4 à 6, de préférence de 1 à 4 parties en poids, le mélange d'excipients comprenant
- un agent de désagrégation
- un agent soluble diluant à propriétés liantes, constitué par un polyol de moins de 13 atomes de carbone se présentant soit sous la forme du produit directement compressible dont le diamètre moyen des particules est de 100 à 500µm, soit sous la forme d'une poudre dont le diamètre moyen des particules est inférieur à 100 µm, ce polyol étant de préférence choisi dans le groupe comprenant le mannitol, le xylitol, le sorbitol et le maltitol, étant entendu que le sorbitol ne peut être utilisé seul et que, dans le cas où l'agent soluble diluant à propriétés liantes est unique, il est utilisé sous la forme du produit directement compressible alors que, dans le cas où il y a au moins deux agents solubles diluants à propriétés liantes, l'un est présent sous la forme directement compressible et l'autre sous la forme poudre, le polyol pouvant alors être le même, les proportions de polyol directement compressible et de polyol poudre étant de 99/1 à 20/80, de préférence de 80/20 à 20/80,
- un lubrifiant,
- un agent perméabilisant, et
- avantageusement des édulcorants, des arômes et des colorants,
la proportion d'agent de désagrégation et d'agent soluble par rapport à la masse du comprimé étant de 1 à 15%, de préférence de 2 à 7% en poids pour le premier et de 30 à 90%, de préférence de 40 à 70% en poids pour le second.

L'agent soluble diluant à propriétés liantes est constitué par un polyol de moins de 13 atomes de carbone se présentant soit sous la forme du produit directement compressible dont le diamètre moyen des particules est compris entre 100 et 500 micromètres, soit sous la forme d'une poudre dont le diamètre moyen des particules est inférieur à 100 micromètres, ce polyol étant de préférence choisi dans le groupe comprenant le mannitol, le xylitol, le sorbitol et le maltitol, le sorbitol ne pouvant être utilisé seul.

Lorsque l'agent soluble diluant à propriétés liantes est unique, donc différent du sorbitol, il est utilisé sous la forme du produit directement compressible.

Lorsqu'on a recours à au moins deux agents solubles diluants à propriétés liantes, l'un est présent sous la forme du produit directement compressible et l'autre, qui peut être constitué par le même polyol, sous la forme d'une poudre dont le diamètre moyen des particules constitutives est inférieur à 100 micromètres, les proportions de polyol directement compressible et de polyol poudre étant de 99/1 à 20/80, de préférence de 80/20 à 20/80.

L'agent de désagrégation est choisi dans le groupe comprenant notamment la carboxyméthylcellulose sodique réticulée désignée dans le métier par le terme croscarmellose, la crospovidone et leur mélange. Grâce au choix et à la proportion de cet agent de désagrégation, le comprimé conserve une dureté acceptable pour des conditions de manipulation normales des comprimés lorsqu'ils sont conservés en conditionnement étanche jusqu'à des températures d'au moins 30°C.

Les proportions respectives d'agent de désagrégation et d'agent soluble retenues pour la constitution de l'excipient sont, par rapport à la masse du comprimé, de 1 à 15% en poids pour le premier et de 30 à 90% en poids pour le second.

Le lubrifiant préférentiellement utilisé dans ce mélange d'excipients est choisi dans le groupe comprenant le stéarate de magnésium, le stéarylfumarate de sodium, l'acide stéarique , le polyoxyéthylèneglycol micronisé (Macrogol 6000 micronisé), et leurs mélanges Il peut être utilisé dans une proportion de 0,05 à 2% par rapport à la masse totale du comprimé.

Comme agent perméabilisant on utilise un composé choisi dans le groupe comprenant notamment des silices ayant une grande affinité pour les solvants aqueux, telles que la silice précipitée plus connue sous le nom de marque Syloïd®, les maltodextrines, les β-cyclodextrines et leurs mélanges.

L'agent perméabilisant permet la création d'un réseau hydrophile qui facilite la pénétration de la salive et contribue ainsi à une meilleure désagrégation du comprimé.

Selon un mode de réalisation tout à fait avantageux des comprimés conformes à l'invention, l'agent perméabilisant est la silice précipitée plus connue sous le nom de marque Syloïd® FP244. En effet, non seulement cette silice contribue à une meilleure désagrégation des comprimés mais en outre, de par ses propriétés d'agent d'écoulement, elle favorise les réarrangements particulaires au cours de la compression et elle permet de réduire , d'une part, la quantité de lubrifiant hydrophobe nécessaire pour assurer la fabrication dans des conditions optimales et, d'autre part, de réduire l'intensité de la force de compression pour obtenir un comprimé manipulable dans ces conditions industrielles.

La proportion d'agent perméabilisant par rapport à la masse du comprimé est comprise entre 0,5 et 5% an poids.

On fait comporter également au mélange d'excipients entrant dans la composition des comprimés selon l'invention un édulcorant, et éventuellement un arôme et un colorant.

L'édulcorant peut être choisi dans le groupe comprenant notamment l'aspartam, l'acesulfame de potassium, le saccharinate de sodium, la néohespéridine dihydrochalcone, et leurs mélanges.

Les arômes et colorants sont ceux utilisés habituellement en pharmacie pour la préparation de comprimés.

Les comprimés conformes à l'invention présentent, par rapport aux comprimés du genre en question qui existent déjà, une amélioration, de la palatabilité et en particulier du goût et de la texture et peuvent permettre de réduire le ratio masse du comprimé/dose de principe actif.

Ils présentent une dureté satisfaisante permettant leur manipulation dans des conditions opératoires standards sans précautions opératoires particulières. A titre indicatif, on signale que des duretés répondant à ces conditions sont généralement comprises entre 20 et 70 Newtons.

Les comprimés conformes à l'invention peuvent être préparés de la façon suivante ou par tout autre procédé approprié. On ajoute des particules de principe actif enrobé présentant des caractéristique intrinsèques de compression à un mélange d'excipients comportant un agent de désagrégation, un agent soluble diluant à propriétés liantes, et un agent perméabilisant, et avantageusement un lubrifiant, des édulcorants, des arômes et des colorants, dans les proportions indiquées ci-dessus. Le mélange ainsi obtenu est soumis à une homogénéisation dans un mélangeur à sec. Le mélange est ensuite soumis à une force de compression qui confère au comprimé résultant une dureté satisfaisante permettant l'industrialisation de sa fabrication et sa manipulation dans des conditions normales sans précautions opératoires particulières; à titre indicatif, on signale que des duretés répondant à ces conditions sont généralement comprises entre 20 et 70 Newtons.

### EXEMPLES

### EXEMPLE 1.

### Comprimé d'ibuprofène dosé à 200 mg

Le tableau I donne la formule unitaire et la formule centésimale de ce comprimé.

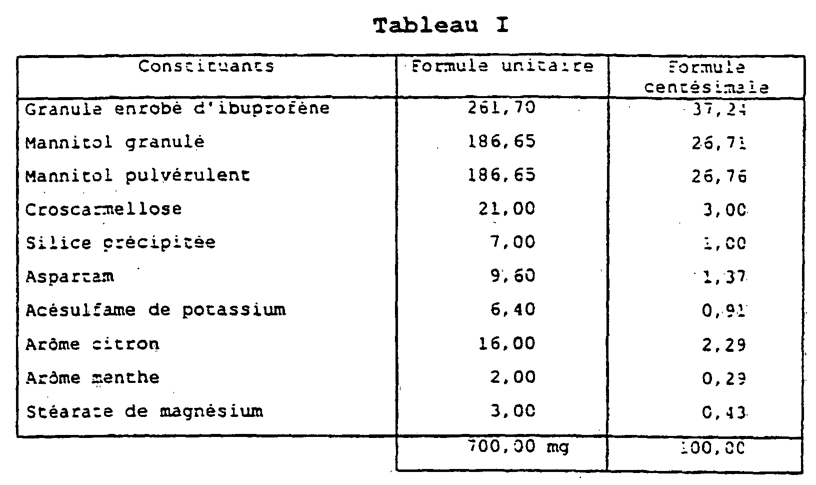

Ce comprimé est préparé comme indiqué ci-après.

On calibre les excipients identifiés dans le tableau I sur une grille de 1000µm d'ouverture de mailles.

On pèse les différents constituants dans des récipients séparés de contenance adaptée.

On introduit dans un mélangeur par retournement des particules enrobées d'ibuprofène (dont la formule est donnée dans le Tableau II ci-après), le mannitol granulé, le mannitol pulvérulent, la croscarmellose, l'aspartam, l'acésulfame de potassium, la silice précipitée et les arômes.

On prépare un mélange homogène.

On arrête le mélangeur et on ajoute le stéarate de magnésium et on poursuit l'opération de mélange pendant 1 à 5 min suivant la masse du mélange.

On comprime le mélange obtenu sur une machine rotative afin d'obtenir des comprimés ayant le caractéristiques suivantes :
- masse moyenne comprise entre 665mg et 735mg;
- résistance à la rupture comprise entre 20 et 50 N ;
- temps de désagrégation moyen en bouche inférieur à 40 secondes.

Ce temps de désagrégation correspond à la durée qui sépare, d'une part, le moment de la mise en place du comprimé dans la bouche au contact de la salive et, d'autre part, le moment de la déglutition de la suspension résultant de la désagrégation du comprimé au contact de la salive.

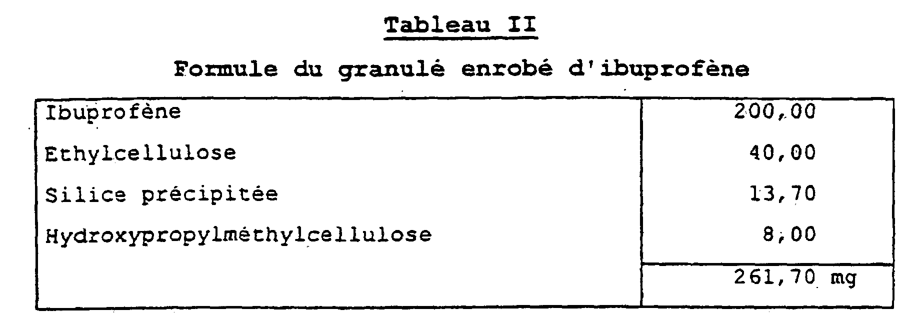

### EXEMPLE 2: Comprimé d'aspirine dosé à 500mg

Le tableau III donne la formule unitaire et la formule centésimale de ce comprimé.

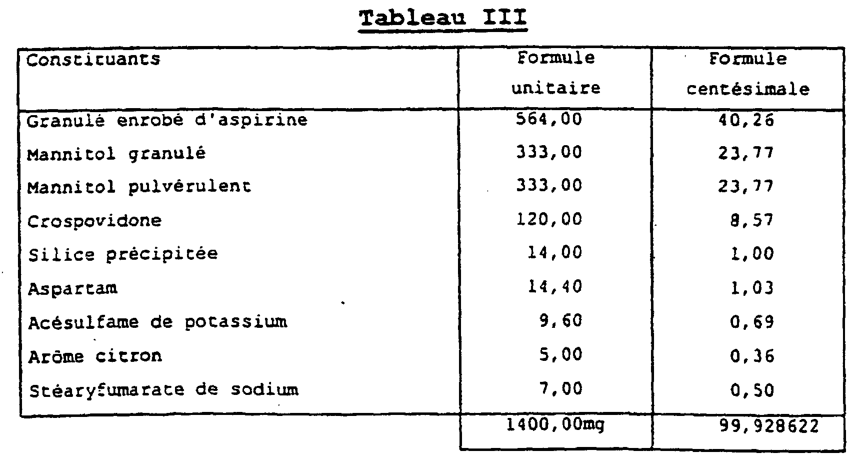

Les comprimés sont préparés de la même façon que dans l'exemple 1, à l'aide de granulés enrobés ayant la formule donnée dans le tableau IV ci-après.

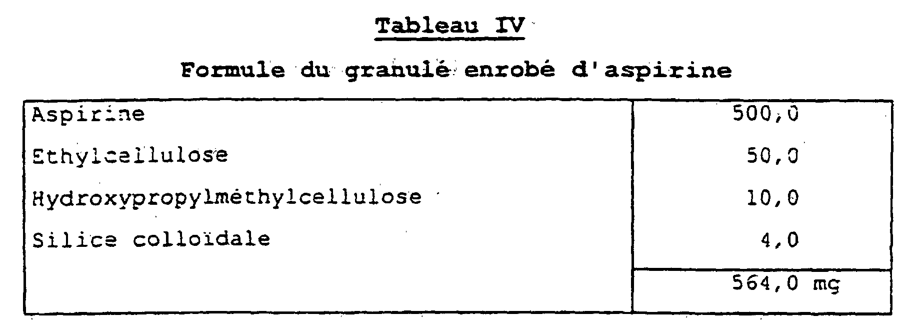

### EXEMPLE 3 : Comprimé de paracétamol dosé à 500mg

Le tableau V donne la formule unitaire et la formule centésimale de ce comprimé.

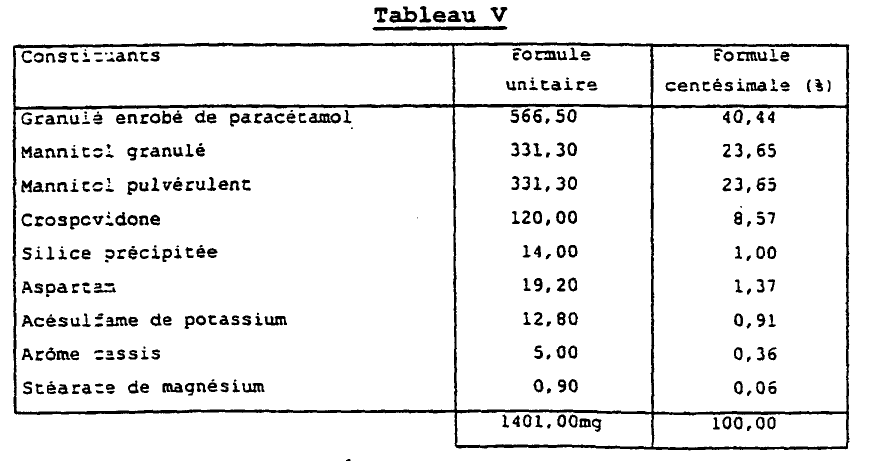

Les comprimés sont préparés de la même façon que dans l'exemple 1 à l'aide de granulés enrobés ayant la formule donnée dans le tableau VI ci-après.

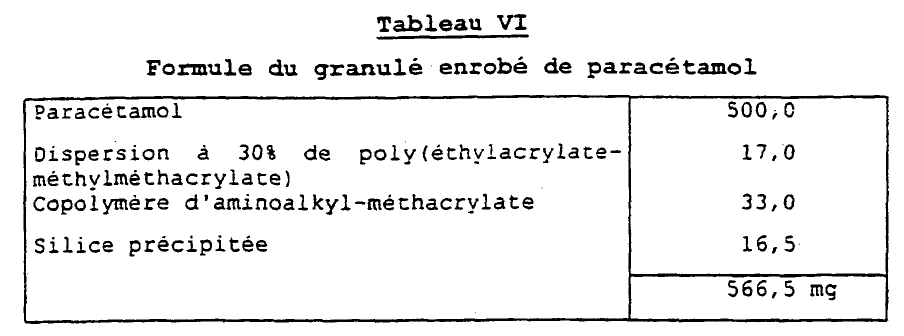

## Revendications

1. Comprimé multiparticulaire perfectionné se désagrégeant dans la bouche au contact de la salive en moins de 40 secondes, **caractérisé par le fait qu'**il est à base de particules de principe actif enrobé, lesdites particules présentant des caractéristiques intrinsèques de compression et d'un mélange d'excipients, la proportion de mélange d'excipients par rapport aux particules de principe actif enrobé étant de 0,4 à 6, de préférence de 1 à 4 parties en poids, le mélange d'excipients comprenant
- un agent de désagrégation
- un agent soluble diluant à propriétés liantes, constitué par un polyol de moins de 13 atomes de carbone se présentant soit sous la forme du produit directement compressible dont le diamètre moyen des particules est de 100 à 500µm, soit sous la forme d'une poudre dont le diamètre moyen des particules est inférieur à 100 µm, ce polyol étant de préférence choisi dans le groupe comprenant le mannitol, le xylitol, le sorbitol et le maltitol, étant entendu que le sorbitol ne peut être utilisé seul et que, dans le cas où l'agent soluble diluant à propriétés liantes est unique, il est utilisé sous la forme du produit directement compressible alors que, dans le cas où il y a au moins deux agents solubles diluants à propriétés liantes, l'un est présent sous la forme directement compressible et l'autre sous la forme poudre, le polyol pouvant alors être le même, les proportions de polyol directement compressible et de polyol poudre étant de 99/1 à 20/80, de préférence de 80/20 à 20/80,
- un lubrifiant,
- un agent perméabilisant, et
- avantageusement des lubrifiants, des édulcorants, des arômes et des colorants, la proportion d'agent de désintégration et d'agent soluble par rapport à la masse du comprimé étant de 1 à 15%, de préférence de 2 à 7% en poids pour le premier et de 30 à 90%, de préférence de 40 à 70% en poids pour le second.

2. Comprimé selon la revendication 1 **caractérisé par le fait que** le principe actif est choisi dans le groupe comprenant notamment l'aspirine, le paracétamol et l'ibuprofène.

3. Comprimé selon la revendication 1 ou la revendication 2 **caractérisé par le fait que** l'agent de désagrégation est choisi dans le groupe comprenant notamment la croscarmellose, la crospovidone et leur mélange.

4. Comprimé selon l'une des revendications 1 à 3, **caractérisé par le fait que** l'agent perméabilisant est choisi dans le groupe comprenant les silices présentant une grande affinité pour les solvants aqueux, telles que la silice précipitée, les maltodextrines, les β-cyclodextrines et leurs mélanges.

5. Comprimé selon l'une des revendications 1 à 4, **caractérisé par le fait que** l'agent perméabilisant est la silice précipitée.

6. Comprimé selon l'une des revendications 1 à 5, **caractérisé par le fait que** la proportion d'agent perméabilisant par rapport à la masse du comprimé est de 0,1 à 10%, et de préférence de 0,5 à 5%.

7. Comprimé selon l'une quelconque des revendications 1 à 6, **caractérisé par le fait que** le lubrifiant est choisi dans le groupe comprenant notamment le stéarate de magnésium, le stéarylfumarate de sodium, l'acide stéarique, le polyoxyéthylèneglycol micronisé, et leurs mélanges.

8. Comprimé selon l'une des revendications 1 à 7, **caractérisé par le fait que** l'édulcorant est choisi dans le groupe comprenant notamment l'aspartam, l'acesulfame de potassium, le saccharinate de sodium, la néohespéridine dihydrochalcone, et leurs mélanges.

## Patentansprüche

1. Verbesserte Multipartikel-Tablette, die sich im Mund bei Kontakt mit dem Speichel in weniger als 40 Sekunden auflöst,
**dadurch gekennzeichnet,**
**dass** sie aus Partikeln von umhülltem Wirkstoff, wobei diese Partikel intrinsische Presseigenschaften aufweisen, sowie aus einem Gemisch von Grundmassen hergestellt ist,
wobei das Verhältnis des Gemischs von Grundmassen zu den Wirkstoff-Partikeln bei 0,4 bis 6, vorzugsweise bei 1 bis 4 Gewichtsanteilen liegt, wobei das Gemisch von Grundmassen aufweist:
- ein Auflösungsmittel,
- ein lösbares Streckmittel mit Bindungseigenschaften, das von einem Polyalkohol mit weniger als 13 Kohlenstoffatomen gebildet wird und entweder in der Form des direkt komprimierbaren Produkts mit einem durchschnittlichen Durchmesser der Partikel von 100 bis 500 µm oder als ein Pulver mit einem durchschnittlichen Durchmesser der Partikel von weniger als 100 µm vorliegt, wobei dieser Polyalkohol vorzugsweise aus einer Gruppe gewählt wird, die Mannit, Xylit, Sorbit und Maltit umfasst, wobei sich versteht, dass Sorbit nur allein verwendet werden kann und dass das lösbare Streckmittel mit Bindungseigenschaften, falls es allein verwendet wird, in der Form des direkt komprimierbaren Produkts verwendet wird, während in dem Fall, dass mindestens zwei lösbare Streckmittel mit Bindungseigenschaften vorhanden sind, das eine in der direkt komprimierbaren Form und das andere als Pulver vorliegt, wobei der Polyalkohol dann der gleiche sein kann, wobei die Anteile von direkt komprimierbarem Polyalkohol und von pulverförmigem Polyalkohol 99/1 bis 20/80 betragen, und vorzugsweise 80/20 bis 20/80 betragen,
- ein Gleitmittel,
- ein Mittel, das durchlässig macht, sowie
- vorteilhafterweise Gleitmittel, Süßstoffe, Aromastoffe und Farbstoffe,
wobei der Anteil des Auflösungsmittels und des lösbaren Mittels an der Masse der Tablette fiir ersteres 1 bis 15 % und vorzugsweise 2 bis 7 % Gewichtsanteile und für zweiteres 30 bis 90 % und vorzugsweise 40 bis 70 % Gewichtsanteile beträgt.

2. Tablette nach Anspruch 1,
**dadurch gekennzeichnet,**
**dass** der Wirkstoff aus einer Gruppe gewählt wird, die insbesondere Aspirin, Paracetamol und Ibuprofen umfasst.

3. Tablette nach Anspruch 1 oder 2,
**dadurch gekennzeichnet,**
**dass** das Auflösungsmittel aus einer Gruppe gewählt wird, die insbesondere Croscannellose, Crospovidon und deren Gemisch umfasst.

4. Tablette nach einem der Ansprüche 1 bis 3,
**dadurch gekennzeichnet,**
**dass** das durchlässig machende Mittel unter den Kieselsäureanhydriden gewählt wird, die eine große Affinität zu wässerigen Lösungsmitteln aufweisen, wie beispielsweise dem ausgefällten Kieselsäureanhydrid, Maltose-Dextrine, β-Cyclodextrine und ihren Gemischen.

5. Tablette nach einem der Ansprüche 1 bis 4,
**dadurch gekennzeichnet,**
**dass** es sich bei dem durchlässig machenden Mittel um ausgefälltes Kieselsäureanhydrid handelt.

6. Tablette nach einem der Ansprüche 1 bis 5,
**dadurch gekennzeichnet,**
**dass** der Anteil des durchlässig machenden Mittels im Verhältnis zur Masse der Tablette 0,1 bis 10 % und vorzugsweise 0,5 bis 5 % beträgt.

7. Tablette nach einem der Ansprüche 1 bis 6,
**dadurch gekennzeichnet,**
**dass** das Gleitmittel aus einer Gruppe gewählt wird, die insbesondere Natriumstearylfumarat, Stearinsäure, mikronisiertes Polyoxyethylenglykol und deren Gemische umfasst.

8. Tablette nach einem der Ansprüche 1 bis 7,
**dadurch gekennzeichnet,**
**dass** der Süßstoff aus einer Gruppe gewählt wird, die insbesondere Aspartam, Acesulfam-K, Na-Saccharin, Neohesperidindihydrochalkon und deren Gemische umfasst.

## Claims

1. Improved multiparticulate tablet which disintegrates in contact with the saliva in the mouth in less than 40 seconds, **characterized in that** it is based on particles of coated active principle which have intrinsic compression characteristics, and on a mixture of excipients, the ratio of excipient mixture to coated active principle particles being 0.4 to 6 parts by weight, preferably 1 to 4 parts by weight, the mixture of excipients comprising:
- a disintegration agent;
- a soluble diluent agent with binding properties which consists of a polyol having less than 13 carbon atoms and being either in the form of the directly compressible product with an average particle diameter of 100 to 500 µm, or in the form of a powder with an average particle diameter of less than 100 µm, this polyol preferably being selected from the group comprising mannitol, xylitol, sorbitol and maltitol, it being understood that sorbitol cannot be used on its own and that, in the case where there is only one soluble diluent agent with binding properties, it is used in the form of the directly compressible product, whereas in the case where there are at least two soluble diluent agents with binding properties, one is present in the directly compressible form and the other is present in powder form, it then being possible for the polyols to be the same, the ratio of directly compressible polyol to powder polyol being 99/1 to 20/80, preferably 80/20 to 20/80;
- a lubricant;
- a permeabilizing agent; and
- advantageously lubricants, sweeteners, flavourings and colours, the proportion of disintegration agent being 1 to 15% by weight, preferably 2 to 7% by weight, and the proportion of soluble agent being 30 to 90% by weight, preferably 40 to 70% by weight, based in each case on the weight of the tablet.

2. Tablet according to Claim 1, **characterized in that** the active principle is selected from the group comprising especially aspirin, paracetamol and ibuprofen.

3. Tablet according to Claim 1 or Claim 2, **characterized in that** the disintegrating agent is selected from the group comprising especially croscarmellose, crospovidone and mixtures thereof.

4. Tablet according to one of Claims 1 to 3, **characterized in that** the permeabilizing agent is selected from the group comprising silicas with a high affinity for aqueous solvents, such as precipitated silica, maltodextrins, β-cyclodextrins and mixtures thereof.

5. Tablet according to one of Claims 1 to 4, **characterized in that** the permeabilizing agent is precipitated silica.

6. Tablet according to one of Claims 1 to 5, **characterized in that** the proportion of permeabilizing agent is 0.1 to 10%, preferably 0.5 to 5%, based on the weight of the tablet.

7. Tablet according to any one of Claims 1 to 6, **characterized in that** the lubricant is selected from the group comprising especially magnesium stearate, sodium stearyl fumarate, stearic acid, micronized polyoxyethylene glycol and mixtures thereof.

8. Tablet according to one of Claims 1 to 7, **characterized in that** the sweetener is selected from the group comprising especially aspartame, potassium acesulfame, sodium saccharinate, neohesperidin dihydrochalcone and mixtures thereof.
